Europäisches Patentamt

**European Patent Office**

**Office européen des brevets**

(19)

(11) Veröffentlichungsnummer: **0 079 490**

**A1**

## (12) EUROPÄISCHE PATENTANMELDUNG

(21) Anmeldenummer: **82109834.0**

(22) Anmeldetag: **25.10.82**

(51) Int. Cl.$^3$: **C 07 C 47/055**

(30) Priorität: **04.11.81 DE 3143704**

(43) Veröffentlichungstag der Anmeldung:
**25.05.83 Patentblatt 83/21**

(84) Benannte Vertragsstaaten:
**AT BE DE FR GB NL**

(71) Anmelder: **BAYER AG**
**Zentralbereich Patente, Marken und Lizenzen**
**D-5090 Leverkusen 1, Bayerwerk(DE)**

(72) Erfinder: **Schenke, Bernd-Ulrich, Ing.-grad.**
**Fernewaldstrasse 253**
**D-4250 Bottrop(DE)**

(72) Erfinder: **Thiel, Reinhard, Dr.**
**Buschstrasse 215a**
**D-4150 Krefeld(DE)**

(72) Erfinder: **Bauer, Dieter, Dr.**
**Bodelschwinghstrasse 127**
**D-4150 Krefeld(DE)**

(72) Erfinder: **Holm, Reimer, Dr.**
**Am Katterbach 32**
**D-5060 Bergisch-Gladbach 2(DE)**

(54) Verfahren zur Herstellung von Formaldehyd.

(57) Formaldehyd wird hergestellt durch oxidierende Dehydrierung von Methanol bei erhöhter Temperatur in Anwesenheit eines Silberkatalysators, dem Silberoxide und/oder Silbercarbonat zugesetzt wurden.

EP 0 079 490 A1

BAYER AKTIENGESELLSCHAFT      5090 Leverkusen, Bayerwerk

Zentralbereich      Bg/bc/c

Patente, Marken und Lizenzen

## Verfahren zur Herstellung von Formaldehyd

Die Erfindung betrifft ein Verfahren zur Herstellung von Formaldehyd durch oxidierende Dehydrierung von Methanol in Gegenwart eines Silberkatalysators bei erhöhter Temperatur.

Die Herstellung von Formaldehyd im technischen Maßstab durch oxidierende Dehydrierung von Methanol in Gegenwart eines Silberkatalysators ist bekannt und wird z.B. in Ullmanns Enzyklopädie der technischen Chemie, Band 7, S. 659 ff beschrieben. Als Silberkatalysatoren können Schüttungen von Silberkristallen mit gleicher oder unterschiedlicher Korngrößenverteilung (Ein- oder Mehrschichtenkatalysator) oder auch Silberdrahtnetze verwendet werden (vgl. z.B. FIAT-Berichte Nr. 999, Seiten 1 bis 3, BIOS-Bericht Nr. 978, Seiten 2 bis 6, DE-PS 1 231 229, DE-AS 1 294 360 und DE-PS 1 285 995).

Die bislang eingesetzten Silberkatalysatoren haben jedoch den Nachteil, daß sie zu Beginn (Anspringen) der Reaktion durch eine externe Heizquelle vorgeheizt werden müssen, was zu erhöhten Anlage-, Energie- und Betriebskosten führt. So muß beispielsweise der in der

Le A 21 369 -Ausland

DE-PS 1 285 995 beschriebene Mehrschichtenkatalysator zum Anspringen der Reaktion durch eine Heizquelle auf mindestens 400°C vorgeheizt werden (s. Spalte 3, Zeilen 40-42) und der in der DE-AS 2 322 757 beanspruchte drei oder mehr Schichten Silberkristalle aufweisende Katalysator muß zum Anspringen der Reaktion durch eine externe Heizquelle auf 280 bis 300°C vorgeheizt werden (vgl. Spalte 3, Zeilen 44-50).

Um die aufgeführten Nachteile zu umgehen, hat man versucht, durch Zusätze von feinverteiltem Silber die zum Anspringen der Reaktion notwendige Katalysatortemperatur (Anspringtemperatur) zu erniedrigen. Nach der japanischen Patentanmeldung 74/24889 gelingt es durch Zugabe von Silber der Korngröße 40 bis 90 mesh (167 bis 380μm) zum Katalysator, die Anspringtemperatur auf 250°C zu senken. In der DE-AS 2 520 219 wird außerdem beschrieben, daß ein Mehrschichten-Silberkatalysator, dem ein gewisser Anteil an feinverteiltem Silber der Korngröße von 0,001 bis 10μm und der Gesamtoberfläche von 3 bis 30 $m^2$/g feinverteiltem Silber zugesetzt wird, nur auf 180 bis 240°C vorgeheizt werden muß, damit die Reaktion anspringt (s. Spalte 4, Zeilen 7-12). Auf den Einsatz zusätzlicher Beheizungsanlagen kann jedoch auch bei der Verwendung der in der japanischen Patentanmeldung und in der DE-AS 2 520 219 beschriebenen Katalysatoren nicht verzichtet werden, so daß die Verfahren in ihrer Wirtschaftlichkeit noch stark beeinträchtigt sind.

Es wurde nun ein Verfahren zur Herstellung von Formaldehyd durch oxidierende Dehydrierung von Methanol

Le A 21 369

in Anwesenheit eines Silberkatalysators bei erhöhter Temperatur gefunden, das dadurch gekennzeichnet ist, daß man die Umsetzung mit einem Katalysator durchführt, dem Silberoxide und/oder Silbercarbonat zugesetzt wurden.

Als Silberoxide können alle bekannten Silberoxide (vgl. z.B. Gmelin, Handbuch der anorganischen Chemie, Silber ($B_3$) Seite 32) dem Katalysator zugesetzt werden. Bevorzugt kommen als Silberoxide Silber-(I)-oxid ($Ag_2O$), Silber-(II)-oxid ($AgO$) sowie Silber-(III)-oxid ($Ag_2O_3$), besonders bevorzugt Silber-(I)-oxid, in Frage. Die Silberoxide können sowohl einzeln als auch im Gemisch untereinander eingesetzt werden.

Ebenso kann Silbercarbonat selbst oder eine Mischung von Silbercarbonat mit den Silberoxiden oder das im Gmelin, Handbuch der anorganischen Chemie, Silber ($B_3$), Seiten 283 und 284 beschriebene, bei der Alterung von Silbercarbonat entstehende basische Silbercarbonat dem Katalysator zugegeben werden. Auch ist die Verwendung von Mischungen aus Silberoxiden und/oder Silbercarbonat mit anderen feinkörnigen, sauerstoffhaltigen Silberverbindungen, wie Silbernitrit und/oder Silberformiat, möglich.

Die zugesetzten Mengen an Silberoxiden und/oder Silbercarbonat können in weiten Bereichen schwanken. Die dem verwendeten Silberkatalysator zuzusetzende Mindest-

Le A 21 369

menge liegt bei etwa 50 g, vorzugsweise bei 100 bis 2500 g, besonders bevorzugt bei 200 bis 1200 g, pro $m^2$ Katalysatorbettfläche.

Je nach Art des in das erfindungsgemäße Verfahren eingesetzten Silberkatalysators kann der Zusatz an Silberoxiden und/oder Silbercarbonaten bereits bei der Herstellung des Katalysators mit den Silberkristallen vermischt werden oder er kann, vorzugsweise als dünne, gleichmäßige Auflageschicht, auf eine oder mehrere Schichten aufgestreut werden. In einer bevorzugten Ausführungsform werden die verwendeten Silberverbindungen in Form einer dünnen, gleichmäßigen Schicht auf die oberste, dem Anströmgas zugewandte Schicht eines Mehrschichtenkatalysators gestreut.

Die Herstellung von für das beanspruchte Verfahren geeigneten Silberverbindungen kann nach den bekannten Methoden, wie sie z.B. in Gmelin, Handbuch der anorganischen Chemie, Silber ($B_1$) und ($B_3$) beschrieben sind, erfolgen. So kann man beispielsweise Silber-(I)-oxid durch Fällen mit Alkalilauge aus wäßriger Silbersalzlösung oder durch thermische Zersetzung von Silbercarbonat herstellen. Silber-(II)-oxid ist durch Umsetzung von Silbernitrat mit Kaliumperoxodisulfat in neutraler wäßriger Lösung und mit Kaliumpermanganat in alkalischer Lösung darstellbar. Eine einfache Herstellungsmethode für Silbercarbonat ist die Fällung aus wäßriger Silbersalzlösung mit Alkalicarbonat und/oder Alkalihydrogencarbonat. Es ist außerdem möglich, in einer besonders vorteilhaften Ausführungs-

Le A 21 369

form, Silberoxid oder Silbercarbonat durch Lösen eines verbrauchten Silberkatalysators in Salpetersäure und Fällung mit Alkalilauge bzw. mit Alkalicarbonat und/oder Alkalihydrogencarbonat herzustellen.

Als Silberkatalysatoren können alle bekannten Ein- oder Mehrschichtenkatalysatoren, wie sie z.B. in der DE-AS 2 520 219 beschrieben sind, in das erfindungsgemäße Verfahren eingesetzt werden.

Gemäß einer bevorzugten Ausführungsform wird die oxidierende Dehydrierung von Methanol in Gegenwart eines Dreischichten-Silberkatalysators mit folgender Zusammensetzung durchgeführt:

| Korngröße (mm) | Gewichtsprozent des gesamten Katalysators |
|---|---|
| 0,15-0,5 | 5-15 |
| 0,5-1 | 60-80 |
| 1-3 | 15-25 |

Bei diesem Silberkatalysator befindet sich die Schicht mit den gröberen Teilchen unten und bildet, da das zu oxidierende Methanol-Wasserdampf-Gemisch von oben nach unten geführt wird, den von der Ausströmseite abgewandten Teil des Katalysators.

Die Durchführung der oxidierenden Dehydrierung von Methanol in Gegenwart eines Silberkatalysators ist an sich bekannt und kann in dem üblichen Rahmen, d.h. mit den bekannten Ausgangsmaterialien und in den bekannten Druck- und Temperaturbereichen durchgeführt

Le A 21 369

werden (vgl. hierzu z.B. Ullmanns Enzyklopädie der
technischen Chemie, Band 7, Seiten 659 ff).

Nach einer typischen Ausführungsform des erfindungsgemäßen Verfahrens wird in einem Verdampfer ein Gasgemisch aus beispielsweise 1,8 Mol Luft, 1,4 Mol Wasserdampf und 1 Mol Methanol erzeugt, auf etwa 80 bis 130°C
erhitzt und bei Temperaturen von etwa 550 bis 800°C,
insbesondere bei 650 bis 700°C, am Silberkatalysator umgesetzt. Zur Erhöhung der Ausbeute und der Lebensdauer des
Katalysators ist es vorteilhaft, das zur Herstellung des
beschriebenen Gasgemisches verwendete Gemisch aus Methanol
und Wasser vor der Verdampfung mit einem sauren und gegebenenfalls einem basischen Ionenaustauscher zu behandeln
(vgl. hierzu die deutsche Anmeldung P 31 10 723).

Um Ausbeuteverluste durch thermische Zersetzung des
gebildeten Formaldehyds zu vermeiden, wird das die Reaktionszone verlassende Gasgemisch innerhalb kurzer Zeit
auf mindestens 400°C abgekühlt und anschließend einer
Absorptionsanlage zugeführt, in der Formaldehyd und
nicht-umgesetztes Methanol unter weiterer Kühlung mit
Wasser ausgewaschen werden.

Nach dem erfindungsgemäßen Verfahren wird vor Beginn
der Reaktion der Katalysator auf eine Temperatur von
ca. 110 bis 140°C, vorzugsweise auf 115 bis 130°C,
erhitzt. Dazu verwendet man vorteilhafterweise heiße
Inertgase, wie Stickstoff. Besonders zweckmäßig wird

Le A 21 369

der Katalysator zunächst mit heißen Inertgasen auf die erforderliche Starttemperatur von vorzugsweise 115 bis 130°C vorgewärmt und anschließend mit dem auf etwa gleiche Temperatur erhitzten Ausgangsgemisch beaufschlagt. Dadurch wird die Kondensation von Methanol auf dem kalten Katalysator vermieden sowie das sofortige Einsetzen der Reaktion beim Beaufschlagen mit den Einsatzstoffen und eine Verkürzung der Anfahrphase erreicht. Da der Katalysator nur auf die vorgenannten niedrigen Temperaturen vorgeheizt werden muß, kann die Erzeugung von heißen Inertgasen in den ohnehin zur Überhitzung des Ausgangsgemisches installierten Wärmeaustauschern durchgeführt werden.

Der Beginn der exothermen Reaktion wird durch einen raschen Anstieg der Katalysatortemperatur auf die durch die Zusammensetzung des Ausgangsgemisches vorgegebene Temperatur von etwa 600°C angezeigt. Danach wird durch eine entsprechende Erhöhung der Luftmenge die Katalysatortemperatur auf etwa 600 bis 800°C, vorzugsweise auf 630 bis 700°C, erhöht. Die Temperaturerhöhung kann rasch oder langsam, zweckmäßigerweise innerhalb von ca. 10 bis 60 Minuten, kontinuierlich oder in Intervallen, durchgeführt werden.

Nach der Einstellung der Endtemperatur des Katalysators wird die Zufuhr von Methanol, Wasser, Luft und/oder Inertgasen erhöht und der Katalysator auf diese Weise mit der gewünschten Menge an Ausgangsgemisch belastet. Die Erhöhung der Katalysatorbelastung kann vergleichsweise schnell, z.B. in etwa 2 bis 10 Stun-

Le A 21 369

den, kontinuierlich oder diskontinuierlich erfolgen.

Im Vergleich zu den Verfahren des Standes der Technik kann bei dem erfindungsgemäßen Verfahren auf eine zusätzliche Heizquelle verzichtet werden, wodurch Anlage-, Energie- und Betriebskosten eingespart werden. Darüber hinaus sind die Aufheizzeit und die zum Erreichen der Endbelastung des Katalysators benötigte Zeit kürzer, wodurch Ausbeuteverluste vermieden und in der Anfangsphase höhere Raum/Zeit-Ausbeuten erreicht werden.

Im Hinblick auf den zuvor diskutierten Stand der Technik sind die beschriebenen Vorteile des erfindungsgemäßen Verfahrens überraschend, da man nach dem Stand der Technik hätte erwarten müssen, daß die Aktivität von Silberkatalysatoren mit zunehmender Oberfläche und Feinheit der verwendeten Silberkristalle ansteigt und deshalb Katalysatoren mit einem gewissen Anteil an solchen Silberteilchen schon bei relativ niedrigen Temperaturen anspringen. Die nach dem erfindungsgemäßen Verfahren dem Katalysator zugesetzten Silberoxide können demgegenüber aber relativ grob und von geringer Oberfläche sein. So kann die Oberfläche von Silberoxid beispielsweise nur 1 $m^2$/g oder weniger betragen.

Da das bei der Reaktionstemperatur aus den zugesetzten Silberverbindungen entstehende Silber eine dünne, zusammenhängende Schicht bildet, hätte man außerdem vermuten müssen, daß der Druckverlust am Katalysator erhöht und dadurch die Lebensdauer des Katalysators herab-

Le A 21 369

gesetzt werden, was aber nicht zutrifft.

Die nachfolgenden Beispiele sollen das erfindungsgemäße
Verfahren verdeutlichen. Die in den Beispielen angegebenen Teile bedeuten Gewichtsteile.

Le A 21 369

## Beispiel 1

### 1.1 Herstellung von Silber-(I)-oxid

Eine 1-molare wäßrige Silbernitratlösung wird bei Raumtemperatur unter ständigem Rühren mit der stöchiometrischen Menge Kaliumhydroxid (2-molare, wäßrige Lösung) versetzt. Der ausgefallene Niederschlag wird abgesaugt, mehrmals mit Kohlendioxid-freiem Wasser gewaschen und bei 80°C im Vakuum getrocknet.

### 1.2 Anfahrvorgang und Umsetzung

In einem senkrechten Rohrreaktor wird ein aus Silberkristallen (0,187 Teilen) bestehender Katalysator folgender Zusammensetzung aufgebaut:

|  | Anteil am Katalysator (Gew.-%) | Korngröße (mm) |
|---|---|---|
| Schicht 1 | 80,2 | 1,0 bis 2,5 |
| Schicht 2 | 5,3 | 0,75 bis 1,0 |
| Schicht 3 | 14,5 | 0,2 bis 0,75 |

Auf Schicht 3 wird in Form einer dünnen, gleichmäßigen Schicht feinverteiltes Silber der Korngröße 0,1 bis 1$\mu$m und einer Gesamtoberfläche von 14 $m^2$/g in einer Menge von 1000 g/$m^2$ Katalysatorbettfläche gestreut. Darüber wird ebenfalls in Form einer gleichmäßigen Schicht Silber-I-oxid (Ag$_2$O) in einer Menge von 1000 g/$m^2$

Le A 21 369

Katalysatorbettfläche verteilt.

Der Katalysator entspricht bis auf den Anteil an $Ag_2O$ dem in der deutschen Auslegeschrift 2 520 219 in Beispiel 2 beschriebenen Katalysator.

Das Ausgangsgemisch wird am Kopf des Reaktors zugeführt, so daß es zunächst auf die Silber-(I)-oxid-Schicht trifft.

Der Katalysator wird durch Einleiten von heißem Stickstoff auf 120°C vorgewärmt.

In einem Verdampfer wird eine Mischung von 14 Teilen Methanol und 100 Teilen Wasser auf 75°C erhitzt. Anschließend werden 75 Teile Luft durch die heiße Lösung geleitet. Das dabei entstehende Gemisch aus Methanoldampf, Luft und Wasserdampf wird in einem Überhitzer auf etwa 120 bis 130°C überhitzt und dem Katalysator zugeführt. Die Reaktion beginnt sofort und es stellt sich nach etwa 15 Minuten, entsprechend der Zusammensetzung des Ausgangsgemisches, eine Temperatur von etwa 630°C ein. Bei einer Katalysatortemperatur von mehr als 300°C wird das Ausgangsgemisch nur noch auf etwa 100°C überhitzt.

Innerhalb von 5 Stunden wird die Zufuhr zum Verdampfer auf 3,95 Teile Methanol, 3,03 Teile Wasser und 5,08 Teile Luft/h erhöht und der Katalysator so auf eine Belastung von 1,8 t Methanol/m² Katalysatorbettfläche und Stunde eingestellt. Der Druck im Reaktor steigt

Le A 21 369

- 12 -

dabei auf 1,4 bar. Der entstandene Formaldehyd und nicht-umgesetztes Methanol werden nach dem Abkühlen der Reaktionsgase auf etwa 140°C unter weiterer Kühlung in einer Absorptionsanlage kondensiert und in Wasser gelöst. In Form einer 30,4 %igen Lösung erhält man 3,26 Teile Formaldehyd pro Stunde, entsprechend einer Ausbeute von 88,1 % der Theorie. Der Gehalt an nicht-umgesetztem Methanol beträgt 1,33 % bei einer Ameisensäurekonzentration von 0,005 %.

Gegenüber den in der deutschen Auslegeschrift 2 520 219 aufgeführten Beispielen wird die Anspringtemperatur um 60°C erniedrigt und die zum Einstellen der Endbelastung notwendige Zeit wesentlich verkürzt.

Beispiel 2

Im gleichen Reaktor wie in Beispiel 1 wird ein aus Silberkristallen bestehender Katalysator wie folgt aufgebaut:

|          | Korngröße (mm) | Anteil am Katalysator (Gew.-%) |
|----------|----------------|--------------------------------|
| Schicht 1 | 1-3            | 20                             |
| Schicht 2 | 0,5-1          | 70                             |
| Schicht 3 | 0,15-0,5       | 10                             |

Le A 21 369

Auf Schicht 3 wird Silber-(I)-oxid (Ag$_2$O) in einer Menge von 1000 g/m$^2$ Katalysatorbettfläche gestreut. Die Schichtdicke des Katalysators beträgt etwa 15 mm.

Der Katalysator wird durch Einleiten von heißem Stickstoff auf 115°C vorgewärmt und der Anfahrvorgang analog Beispiel 1 durchgeführt.

Innerhalb von etwa 5 Stunden wird die Endbelastung des Katalysators eingestellt. Dabei werden dem Verdampfer in Form einer wäßrigen Methanollösung (ca. 56 % Methanolgehalt), die zuvor über einen sauren Ionenaustauscher, wie er z.B. im Handel unter der Bezeichnung Lewatit $^R$ SC 108 (H-Form) erhältlich ist, gereinigt wurde, 3,95 Teile Methanol und 3,03 Teile Wasser pro Stunde zugeführt. Gleichzeitig werden in den Verdampfer 5,08 Teile Luft pro Stunde eingeblasen. Der Druck vor dem Katalysator steigt dabei auf 1,4 bar an.

Die Aufarbeitung der Reaktionsgase erfolgt wie im Beispiel 1 beschrieben. Man erhält pro Stunde 3,30 Teile Formaldehyd in Form einer 30,29 %igen Lösung mit einem Gehalt von 1,30 % Methanol und 0,005 % Ameisensäure. Die Ausbeute beträgt dementsprechend 89,2 % der Theorie.

Nach einer Einsatzdauer von 180 Tagen sind Aktivität und Selektivität des Katalysators praktisch unverändert.

Le A 21 369

## Beispiel 3

In der gleichen Anlage wie in Beispiel 1 wird ein Katalysator gemäß Beispiel 2 aufgebaut. Auf Schicht 3 werden 330 g Silber-(I)-oxid ($Ag_2O$) pro $m^2$ Katalysatorbettfläche in Form einer gleichmäßigen Schicht gestreut. Anfahrvorgang und Umsetzung entsprechen Beispiel 1. Die Temperatur des Katalysators bei Beginn der Reaktion beträgt 125°C.

Man erhält in Form einer 30,8 %igen Lösung mit 1,40 % Methanol und 0,005 % Ameisensäure 3,29 Teile Formaldehyd pro Stunde, entsprechend einer Ausbeute von 88,8 % der Theorie.

## Beispiel 4

Analog Beispiel 2 wird mit derselben Menge Silber-(I)-oxid ($Ag_2O$), jedoch gleichmäßig mit den Silberkristallen der Schicht 3 gemischt, angefahren. Bei Beginn der Reaktion beträgt die Katalysatortemperatur 120°C.

Es werden in Form einer 30,4 %igen Lösung, die 1,26 % Methanol und 0,005 % Ameisensäure enthält, pro Stunde 3,28 Teile Formaldehyd entsprechend einer Ausbeute von 88,6 % der Theorie erhalten.

## Beispiel 5

In der gleichen Anlage wie in den zuvor beschriebenen Versuchen wird der in Beispiel 2 beschriebene Kata-

Le A 21 369

lysator aufgebaut. Auf Schicht 1 werden 1000 g Silber-carbonat ($Ag_2CO_3$) pro $m^2$ Katalysatorbettfläche in Form einer gleichmäßigen, dünnen Schicht verteilt. Der Anfahrvorgang und die Umsetzung entsprechen Beispiel 2. Die Temperatur bei Beginn der Reaktion beträgt 120°C. Man erhält pro Stunde 3,32 Teile Formaldehyd in Form einer 30,43 %igen Lösung mit 1,23 % Methanol und 0,005 % Ameisensäure, entsprechend einer Ausbeute von 89,6 % der Theorie.

Beispiel 6

Katalysatoraufbau, Anfahrvorgang und Umsetzung entsprechen Beispiel 5. Die Menge Silbercarbonat beträgt jedoch nur 330 g pro $m^2$ Katalysatorbettfläche. Die Anspringtemperatur beträgt 120°C.

Entsprechend einer Ausbeute von 88,9 % der Theorie erhält man pro Stunde 3,29 Teile Formaldehyd. Die anfallende Lösung enthält neben 31,0 % Formaldehyd 1,30 % Methanol und 0,005 % Ameisensäure.

Beispiel 7

Anfahrvorgang und Umsetzung werden analog Beispiel 5, jedoch mit einer Mischung aus 95 Teilen Silbercarbonat und 5 Teilen Silber-(I)-oxid, durchgeführt.

Die Anspringtemperatur beträgt 120°C. Man erhält eine 30,6 %ige Formaldehydlösung mit 1,30 % Methanol und 0,005 % Ameisensäure. Die Ausbeute beträgt 88,8 % der Theorie.

Le A 21 369

Patentansprüche

1) Verfahren zur Herstellung von Formaldehyd durch oxidierende Dehydrierung von Methanol in Anwesenheit eines Silberkatalysators bei erhöhter Temperatur, dadurch gekennzeichnet, daß man die Umsetzung mit einem Katalysator durchführt, dem Silberoxide und/oder Silbercarbonat zugesetzt wurden.

2) Verfahren nach Anspruch 1, dadurch gekennzeichnet, daß man als Silberoxide Silber-(I)-oxid, Silber-(II)-oxid und/oder Silber-(III)-oxid zusetzt.

3) Verfahren nach Ansprüchen 1 und 2, dadurch gekennzeichnet, daß man Silber-(I)-oxid zusetzt.

4) Verfahren nach Ansprüchen 1 bis 3, dadurch gekennzeichnet, daß man mindestens 50 g Silberoxide und/oder Silbercarbonat pro $m^2$ Katalysatorbettfläche zusetzt.

5) Verfahren nach Ansprüchen 1 bis 4, dadurch gekennzeichnet, daß man 100 bis 2500 g Silberoxide und/oder Silbercarbonat pro $m^2$ Katalysatorbettfläche zusetzt.

6) Verfahren nach Ansprüchen 1 bis 5, dadurch gekennzeichnet, daß man 200 bis 1200 g Silberoxide und/oder Silbercarbonat pro $m^2$ Katalysatorbettfläche zusetzt.

Le A 21 369

7) Verfahren nach Ansprüchen 1 bis 6, dadurch gekennzeichnet, daß man die Silberoxide und/oder das Silbercarbonat im Gemisch mit Silbernitrit und/oder Silberformiat zusetzt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | C 07 C 47/055 |
| X | Chemical Abstracts, Band 63, Nr. 6, September 1965, Columbus, Ohio, USA V.P. FENTSIK et al. "Effect of an external electric field on the catalytic activity of silver-oxide-silver", column 6367 & Poverkhn i Kontaktn. Yavleniya v Poluprov. Sibir sk. Fiz.-Tekhn. Nauchn.-Issled. Inst. pri Tomskom Gos. Univ., 1964, Seiten 190-198 | 1,3 | |
| Y | Chemical Abstracts, Band 85, Nr. 5, 2. August 1976, Columbus, Ohio, USA N.I. PETROVA et al. "Catalytic activity of vanadium pentoxide with silver oxide additives with respect to the oxidation of methanol to formaldehyde", page 343, column 1, abstract no. 32366v & Zhurnal Fizicheskoi Khimii, Band 50, Nr. 3, 1976, Seiten 663-665 | 1,3 | **RECHERCHIERTE SACHGEBIETE (Int. Cl. ³)**<br><br>C 07 C 47/052<br>C 07 C 47/055 |
| D,Y | DE-A-2 520 219 (BASF) * Ansprüche 1, 2 * | 1,3 | |
| A | DE-A-2 418 712 (NISCHNETAGILSKIJ SAWOD PLASTMASS) * Ansprüche 1, 2 * | 1 | |
| | ----- | | |

Der vorliegende Recherchenbericht wurde für alle Patentansprüche erstellt.

| Recherchenort BERLIN | Abschlußdatum der Recherche 01-02-1983 | Prüfer KNAACK M |
|---|---|---|